# EUROPEAN PATENT SPECIFICATION

(11) **EP 1 279 662 B1**
(45) Date of publication and mention of the grant of the patent: **16.08.2006**
(21) Application number: 02079355.0
(22) Date of filing: 08.03.1995
(51) Int. Cl.: C07C 255/46, C07C 253/30

(54) **Process for preparing phenylcyclohexan-1-ylcarboxylic acids**
Verfahren zur Herstellung von Phenylcyclohexan-1-yl Carbonsäuren
Procédé de préparation d'acides phénylcyclohexan-1-yl carboxyliques

(30) Priority: 11.03.1994 GB 9404706
(43) Date of publication of application: 29.01.2003
(62) Divisional of application: 95913622.7
(73) Proprietor: SMITHKLINE BEECHAM CORPORATION, Philadelphia, Pennsylvania 19101-7929 (US)
(72) Inventor: Bordas-Nagy, Joseph, Sunnyvale, CA 94086 (US); Gorycki, Peter, Conshohocken, PA 19428 (US); Webb, Kevin Scott, Phoenixville, PA 19460 (US)
(74) Representative: Quillin, Helen Kaye

(56) References cited:
- WO-A-93/19749
- WO-A-93/19750
- WO-A-93/19751

## Description

### Field of the Invention

The present invention relates to a process for the preparation of phenylcyclohexan-1-ylcarboxylic acids.

### Background of the Invention

Bronchial asthma is a complex, multifactorial disease characterized by reversible narrowing of the airway and hyperreactivity of the respiratory tract to external stimuli. Identification of novel therapeutic agents for asthma is made difficult by the fact that multiple mediators are responsible for the development of the disease. Thus, it seems unlikely that eliminating the effects of a single mediator will have a substantial effect on all components of chronic bronchial asthma.

An alternative to the "mediator approach" is to regulate the activity of cells responsible for the pathophysiology of asthma. Cyclic AMP (cAMP, adenosine cyclic 3',5'-monophosphate) modulates the activity of most, if not all, of the cells that contribute to the pathophysiology of extrinsic (allergic) asthma. An elevation of cAMP would produce beneficial effects including: (1) airway smooth muscle relaxation, (2) inhibition of mast cell mediator release, (3) suppression of neutrophil degranulation, (4) inhibition of basophil degranulation, and (5) inhibition of monocyte and macrophage activation. Cyclic AMP has been shown to mediate cellular responses to a wide range of hormones, neurotransmitters and drugs; [Krebs Endocrinology Proceedings of the 4th International Congress Excerpta Medica, 17-29, 1973].

One potential means to regulate the activity of cells responsible for the pathophysiology of asthma is to control the intracellular levels of cyclic AMP. Cellular cAMP levels are elevated when an appropriate agonist binds to particular cell surface receptors, thereby activating adenylate cyclase to convert Mg⁺²-ATP to cAMP at an accelerated rate. The principal cellular mechanism for the inactivation of cAMP is hydrolysis of the 3'-phosphodiester bond by one or more of a family of isozymes referred to as cyclic nucleotide phosphodiesterases (cyclic nucleotide phosphodiesterase hereinafter "PDE"s). Hence, compounds that activate adenylate cyclase or inhibit phosphodiesterase should be effective in suppressing the inappropriate activation of airway smooth muscle and a wide variety of inflammatory cells.

It has been shown that a distinct PDE isozyme, PDE IV, is responsible for cAMP breakdown in airway smooth muscle and inflammatory cells. [Torphy, "Phosphodiesterase lsozymes: Potential Targets for Novel Anti-asthmatic Agents" in New Drugs for Asthma, Barnes, ed. IBC Technical Services Ltd., 1989]. Research indicates that inhibition of this enzyme not only produces airway smooth muscle relaxation, but also suppresses degranulation of mast cells, basophils and neutrophils along with inhibiting the activation of monocytes and neutrophils. The beneficial effects of PDE IV inhibition are markedly potentiated when adenylate cyclase activity of target cells is elevated by appropriate hormones or autocoids. Thus, PDE IV inhibitors would be effective in the asthmatic lung, where levels of prostaglandin E₂ and prostacyclin (both activators of adenylate cyclase) are elevated. PDE IV inhibitors offer a unique approach to the pharmacotherapy of bronchial asthma, and possess significant therapeutic advantages over agents currently on the market. The compounds of this invention have the ability to inhibit PDE IV.

The compounds of this invention also inhibit the production of TNF, a serum glycoprotein. Excessive or unregulated TNF production has been implicated in mediating or exacerbating a number of undesirable physiological conditions, such as diseases , and including rheumatoid arthritis, rheumatoid spondylitis, osteoarthritis, gouty arthritis and other arthritic conditions; sepsis, septic shock, endotoxic shock, gram negative sepsis, toxic shock syndrome, adult respiratory distress syndrome, cerebral malaria, chronic pulmonary inflammatory disease, silicosis, pulmonary sarcoidosis, bone resorption diseases, reperfusion injury, graft vs. host reaction, allograft rejections, fever and myalgias due to infection, such as influenza, cachexia secondary to infection or malignancy, human acquired immune deficiency syndrome (AIDS), cachexia secondary to AIDS, AIDS related complex (ARC), keloid formation, scar tissue formation, Crohn's disease, ulcerative colitis, or pyresis, in addition to a number of autoimmune diseases, such as multiple sclerosis, autoimmune diabetes and systemic lupus erythematosis.

AIDS results from the infection of T lymphocytes with Human Immunodeficiency Virus (HIV). At least three types or strains of HIV have been identified: HIV-1, HIV-2 and HIV-3. As a consequence of HIV infection, T-cell-mediated immunity is impaired and infected individuals manifest severe opportunistic infections and/or unusual neoplasms. HIV entry into a T lymphocyte requires prior T lymphocyte activation. Once an activated T lymphocyte has been infected with HIV, the T lymphocyte must be maintained in an activated state in order to permit HIV gene expression and/or HIV replication.

Cytokines, including TNF, are implicated in activated T-cell-mediated HIV protein expression and/or virus replication as playing a role in maintaining T lymphocyte activation. Therefore, interference with cytokine activity in an HIV-infected individual, such as by inhibition of TNF production, aids in limiting the maintenance of T cell activation, and thereby mitigates the progression of HIV infection to previously uninfected cells. When HIV infection of previously uninfected cells is diminished, a slowing or elimination of the progression of immune dysfunction caused by HIV infection results.

Monocytes, macrophages, and related cells, such as kupffer and glial cells, have also been implicated in the maintenance of HIV infection. These cells, like T cells, are targets for viral replication, where the level of viral replication is dependent upon the activation state of the cells. [See Rosenberg *et al.,* The Immunopathogenesis of HIV Infection, Advances in Immunology, Vol. 57, 1989]. Monokines, such as TNF, have been shown to activate HIV replication in monocytes and/or macrophages [See Poli *et al.,* Proc. Natl. Acad. Sci., 87:782-784, 1990], therefore, inhibition of monokine production or activity aids in limiting HIV progression as stated above for T cells.

TNF has also been implicated in various roles with other viral infections, such as the cytomegalovirus (CMV), influenza virus, adenovirus, and the herpes virus for similar reasons as those noted. TNF is also associated with yeast and fungal infections. Specifically *Candida albicans* has been shown to induce TNF production *in vitro* in human monocytes and natural killer cells. [See Riipi *et al.,* Infection and Immunity, 58(9):2750-54, 1990; and Jafari *et al.,* Journal of Infectious Diseases, 164:389-95, 1991. See also Wasan *et al.,* Antimicrobial Agents and Chemotherapy, 35(10):2046-48, 1991; and Luke *et al.,* Journal of Infectious Diseases, 162:211-214, 1990].

### Summary of the Invention

This invention relates to a process for preparing an acid of formula (IV) 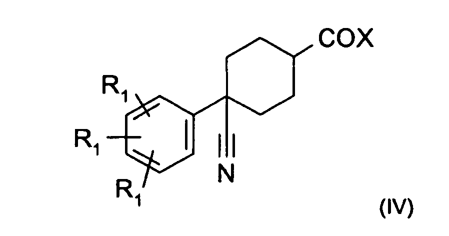 wherein R₁ is hydrogen or another substituent and X is OH or a salt thereof or -O-C₁₋₆alkyl, which process comprises hydrolyzing a thioketene of formula (C) 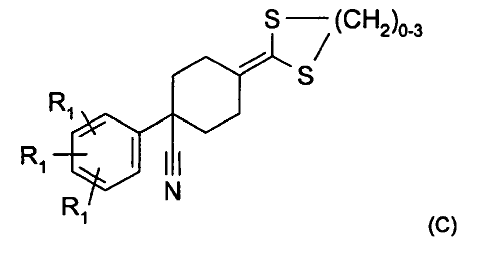 using trifluoroacetic acid and aqueous solvent system and heating the mixture to between 40 and 80°C for 30 minutes to 2 hours, and then adding a base.

An example of a compound of formula (IV) produced by this process is cis-[4-cyano-4-(3-cyclopentyloxy-4-methoxyphenyl)cyclohexane-1-carboxylic acid] or a salt thereof.

### Detailed Description of the Invention

### Definitions

As used herein, the following terms and expressions have the indicated meaning.

"Aryl" or "aralkyl", unless specified otherwise, means an aromatic ring or ring system of 6-10 carbon atoms, such as phenyl, benzyl, phenethyl, or naphthyl. The alkyl chain is meant to include both straight or branched chain radicals of 1 to 4 carbon atoms.

The term "C₁₋₂ alkyl", "C₁₋₄ alkyl", "C₁₋₆ alkyl" or "alkyl groups" includes both straight or branched chain radicals of 1 to 10 carbon atoms, unless the chain length is otherwise limited thereto, including, but not limited to methyl, ethyl, n-propyl, isopropyl, n-butyl, sec-butyl, isobutyl, tert-butyl, and the like.

The term "C₃₋₇ cycloalkyl" means groups of 3-7 carbon atoms, such as cyclopropyl, cyclopropylmethyl, cyclopentyl, or cyclohexyl.

"Halo" includes all halogen radicals, i.e., chloro, fluoro, bromo, or iodo.

"Heteroaryl" means an aromatic ring system containing one or more heteroatoms, such as imidazolyl, triazolyl, oxazolyl, pyridyl, pyrimidyl, pyrazolyl, pyrrolyl, furanyl, or thienyl.

### Example 1

### Preparation of cis-[4-cyano-4-(3-cyclopentyloxy-4-methoxyphenyl)cyclohexane-1-carboxylic acid]

### 1(a) (3-Cyclopentyloxy-4-methoxyphenyl)acetonitrile

To a solution of 3-cyclopentyloxy-4-methoxybenzaldehyde (20 g, 90.8 mmol) in acetonitrile (100 mL) was added lithium bromide (15 g, 173 mmol) followed by the dropwise addition of trimethylsilylchloride (17.4 mL, 137 mmol). After 15 min, the reaction mixture was cooled to 0° C, 1,1,3,3-tetramethyldisiloxane (26.7 mL, 151 mmol) was added dropwise and the resulting mixture was allowed to warm to room temperature. After stirring for 3 h, the mixture was separated into two layers. The lower layer was removed, diluted with methylene chloride and filtered through Celite® . The filtrate was concentrated under reduced pressure, dissolved in methylene chloride and refiltered. The solvent was removed in vacuo to provide a light tan oil. To a solution of this crude a-bromo-3-cyclopentyloxy-4-methoxytoluene in dimethylformamide (160 mL) under an argon atmosphere was added sodium cyanide (10.1 g, 206 mmol) and the resulting mixture was stirred at room temperature for 18 h, then poured into cold water (600 mL) and extracted three times with ether. The organic extract was washed three times with water, once with brine and was dried (K₂CO₃). The solvent was removed in vacuo and the residue was purified by flash chromatography (silica gel, 10% ethyl acetate/hexanes) to provide an off-white solid ( m.p. 32-34° C); an additional quantity of slightly impure material also was isolated.

### 1 (b) Dimethyl 4-cyano-4-(3-cyclopentyloxy-4-methoxyphenyl)pimelate

To a solution of (3-cyclopentyloxy-4-methoxyphenyl)acetonitrile (7 g, 30.3 mmol) in acetonitrile (200 mL) under an argon atmosphere was added a 40% solution of Triton-B in methanol (1.4 mL, 3.03 mmol) and the mixture was heated to reflux. Methyl acrylate (27 mL, 303 mmol) was added carefully, the reaction mixture was maintained at reflux for 5 h and then cooled. The mixture was diluted with ether, was washed once with 1N hydrochloric acid and once with brine, was dried (MgSO₄) and the solvent was removed in vacuo. The solid residue was triturated with 5% ethanol/hexane to provide a white solid (m.p. 81-82° C); an additional quantity was also obtained from the filtrate. Anal. (C₂₂H₂₉NO₆) calcd: C 65.49, H 7.25, N 3.47. found: C 65.47, H 7.11, N 3.49.

### 1(c) 2-Carbomethoxy-4-cyano-4-(3-cyclopentyloxy-4-methoxyphenyl)cyclohexan-1-one

To a suspension of sodium methoxide (350mL, 1.55 mol., 25% w/w in methanol) in toluene (2.45 L) heated to 80° C under a nitrogen atmosphere was added a solution of dimethyl 4-cyano-4-(3-cyclopentyloxy-4-methoxyphenyl)pimelate (350.0 g, 0.87 mol) in toluene (1.05 L) over 10 min. The reaction was heated to 85° C by distilling away 250 mL of solvent and was vigorously stirred under nitrogen for 2 hours. The reaction was cooled to 50° C and was quenched with 3N (aq) HCI (700 mL, 2.1 mol). The organic layer was isolated, was washed once with deionized water (700 mL) and once with brine (700 mL). The organic layer was concentrated via low vacuum distillation to afford crude 2-carbomethoxy-4-cyano-4-(3-cyclopentyloxy-4-methoxyphenyl)cyclohexane-1-one in toluene. This was dissolved in 4.2 L of dimethyl sulfoxide and used in the next step.

### 1(d) 4-Cyano-4-(3-cyclopentyloxy-4-methoxyphenyl)cyclohexan-1-one

To a suspension of sodium chloride (315 g, 5.39 mol) and deionized water (315 mL) was added the dimethyl sulfoxide (4.2 L) solution of 2-carbomethoxy-4-cyano-4-(3-cyclopentyloxy-4-methoxyphenyl)cyclohexane-1-one (323 g, 0.87 mol) and the resulting suspension was heated to 155° C for 1.75 h. The reaction was cooled to 40° C, was quenched into 8 L of iced water (2° C) and was extracted with ethyl acetate (3.5 L). The aqueous layer was isolated and re-extracted with 2.5 L of ethyl acetate. The combined organic extract (6 L) was washed two times with deionized water (2 x 1 L) and once with brine (1 L). The organic layer was isolated and concentrated in vacuo to afford a residue. This residue was dissolved in refluxing isopropanol (500 mL), was cooled to 0° C and held at this temperature for 1 hour. The crystals were isolated by filtration, were washed with 250 mL of isopropanol (0° C), and were dried in a vacuum oven (45° C at 20 inches) to produce 4-cyano-4-(3-cyclopentyloxy-4-methoxyphenyl)cyclohexan-1-one . m.p. 111-112° C; Anal. (C₁₉H₂₃NO₃) calcd: C 72.82, H 7.40, N 4.47; found: C 72.72, H 7.39, N 4.48.

### 1(e) 2-[4-Cyano-4-(3-cyclopentyloxy-4-methoxyphenyl)cyclohexylidene]-1,3-dithiane

To a solution of 2-trimethylsilyl-1,3-dithiane (9.25 mL, 48.7 mmol) in dry tetrahydrofuran (80 mL) at 0° C under an argon atmosphere was added rapidly n-butyllithium (2.5M in hexanes, 19.2 mL, 48 mmol). After 10 min, the mixture was cooled to -78° C and a solution of 4-cyano-4-(3-cyclopentyloxy-4-methoxyphenyl)cyclohexan-1-one (7.53 g, 23 mmol) in tetrahydrofuran (40 mL) was added. After 10 min, aqueous sodium chloride was added, the mixture was allowed to warm to room temperature and was diluted with water. This mixture was combined with the product of three substantially similar reactions conducted on ketone (3.04, 6.01 and 6.1 g, 48.3 mmol total), the combined mixture was extracted three times with methylene chloride, the extract was dried (MgSO₄) and evaporated. Purification by flash chromatography (silica gel, 10% ethyl acetate/hexanes) provided a white solid. m.p. 115-116° C.

### 1(f) cis-[4-Cyano-4-(3-cyclopentyloxy-4-methoxyphenyl)cyclohexane-1-carboxylic acid]

To a suspension of 2-[4-cyano-4-(3-cyclopentyloxy-4-methoxyphenyl)cyclo-hexylidene]-1,3-dithiane ( 140.0 g, 0.34 mol) in acetonitrile (500 mL) and deioinized water (140 mL) under nitrogen was added trifluoroacetic acid (136 g, 1.19 mol). The suspension was heated to 65° C for 1.25 h followed by the addition of 20% sodium hydroxide (420 g, 2.1 mol). The solution was heated at 70 to 75° C for an additional 1.25 h, was cooled to 45° C, deionized water (420 mL)was added followed by 3N (aq) HCl (392 mL, 1.18 mol). The suspension was cooled to 5° C and held for 1 h. The suspension was filtered, was washed with cold (5° C) deionized water ( 200 mL), and was dried in a vacuum oven (40°C at 20 inches) to obtain crude cis-[4-cyano-4-(3-cyclopentyloxy-4-methoxyphenyl)cyclohexane-1-carboxylic acid]. This material was assayed at 98.5% and was found to a 98.8:1.2 mixture of cis-to-trans isomers, which was contaminated with 0.1% of residual 1,3-propanedithiol. This material was purified via an oxidative workup as follows.

To a hot solution (65° C) of crude cis-[4-cyano-4-(3-cyclopentyloxy-4-methoxyphenyl)cyclohexane-1-carboxylic acid] (85 g, 0.247 mol) in acetonitrile (425 mL) was added 1 M sodium hydroxide ( 425 mL, 0.425 mol). To the solution (60° C) was added 4.25 g of calcium hypochlorite and the suspension was vigorously stirred for 2 h. The reaction was concentrated by distilling out 320 mL of solvent, followed by the addition of ethyl acetate (425 mL). The reaction was again concentrated by distilling out 445 mL of solvent, was cooled to 55° C followed by the addition of ethyl acetate (1.0 L) and 6N (aq.) HCI (100 mL). The organic layer was isolated, was washed three times with deionized water (3 x 300 mL), was filtered and was concentrated by distilling out 530 mL of solvent. To the solution was added ethyl acetate (635 mL) with continued distillation to remove 750 mL of solvent. The solution was cooled to 65° C followed by the addition of hexane ( 340 mL). The suspension was cooled to 5° C, held at this temperature for 1 hour, was filtered and was washed with cold (5° C) 10% ethyl acetate/ hexane ( 200 mL). The solid was collected and was dried in a vacuum oven (40° C at 20 inches) to obtain cis-[4-cyano-4-(3-cyclopentyloxy-4-methoxyphenyl)cyclohexane-1-carboxylic acid]. This material was found to contain no trans isomer. Anal.(C₂₀H₂₅NO₄) calcd: C 69.95, H 7.34, N 4.08; found: C 69.90, H 7.35, N 4.02.

### Reference Example 2

### Preparation of cis-{4-cyano-4-[3-(trans-3-hydroxycyclopentyloxy)-4-methoxyphenyl]-cyclohexane-1-carboxylic acid}

### 2(a) cis-[4-Cyano-4-(3-hydroxy-4-methoxyphenyl)cyclohexane-1-carboxylic acid]

To a solution of boron tribromide in dichlorormethane (0.1 M, 335 mL, 33.5 mmol) under an argon atmosphere at -78° C was slowly added a solution of cis-[4-cyano-4-(3-cyclopentyloxy-4-methoxyphenyl)cyclohexane-1-carboxylic acid] (4.03 g, 11.7 mmol) in dichloromethane (180 mL). The mixture was stirred for 5 min, 15% sodium methoxide in methanol was added to pH 8-9 and the reaction was warmed to RT. Water (100mL) was added and the mixture was acidified with 3N aqueous hydrochloric acid to pH 1-2. The organic layer was separated, was dried (MgSO₄/Na₂SO₄), was filtered and was evaporated. The residue was twice dissolved in chloroform and the solution was evaporated to yield a white solid. ¹H NMR(400 MHz, CDCI₃) □ 7.01 (d, J=2.4 Hz, 1H), 6.96 (d of d, J=2.4, 8.5 Hz, 1H), 3.89 (s, 3H), 2.31 (m, 1 H), 2.21 (br t, J=13.6 Hz, 4H), 1.98 (m,2H), 1.77 (m, 2H); mp 190-193° C.

### 2(b) Methyl cis-[-4-cyano-4-(3-hydroxy-4-methoxyphenyl)cyclohexane-1-carboxylate]

*p*-Toluenesulfonic acid monohydrate (0.015 g, 0.08 mmol) was added to a solution of the compound of Example 2(a) (0.70 g, 2.54 mmol) in dry methanol (20 mL) under an argon atmosphere and the reaction was stirred for 6 h at 45-50° C. The reaction was cooled to RT and was stirred for an additional 16 h. The solution was evaporated and the residue was purified by flash chromatography (silica gel, 50% hexane/ethyl acetate) to yield the title compound as a white solid. ¹H NMR(400 MHz, CDCl₃) □ 7.01 (m, 2H), 6.85 (d, J=9.1 Hz, 1H), 3.90 (s, 3H), 3.72 (s, 3H), 2.35 (t of t, J=3.6, 12.2 Hz, 1 H), 2.14-2.25 (m, 4H), 2.00 (app q, J=13.4 Hz, 1H), 1.99 (app q, J=13.4 Hz, 1 H), 1.77 (app t, J=13.4 Hz, 1 H), 1.76 (app t, J=13.4 Hz, 1 H); mp 106-107° C.

### 2(c) Methyl cis-{-4-cyano-4-[3-(trans-3-hydroxycyclopentyloxy)-4-methoxyphenyl]-cyclohexane-1-carboxylate}

The compound of Example 2(b) (0.69 g, 2.37 mmol) was dissolved in tetrahydrofuran (20 mL) under an argon atmosphere and was treated with triphenylphosphine (1.24 g, 4.74 mmol) and cis-1,3-cyclopentanediol (0.49 g, 4.74 mmol). Diethyl azodicarboxylate (0.83 g, 4.74 mmol) was added and the mixture was stirred at RT for 16 h. The solution was evaporated, the residue was diluted with ether and the white solid was removed by filtration. The filtrate was concentrated and the residue was purified by flash chromatography (silica gel, 50% hexane/ethyl acetate) to yield a mixture of the title compound and triphenylphosphine oxide. The mixture was diluted with ether and the white solid triphenylphosphine oxide was removed by filtration. Evaporation of the filtrate yielded the title compound as a sticky, colorless semi-solid. ¹H NMR(400 MHz, CDCl₃) □ 7.07 (d, J=2.4 Hz, 1H), 7.02 (d of d, J=2.4, 8.8 Hz, 1H), 6.87 (d, J=8.8 Hz, 1H), 4.99 (m, 1H), 4.37 (m, 1H), 3.85 (s, 3H), 3.74 (s, 3H), 3.16 (d, J=9.1 Hz, 1 H), 2.39 (m, 1 H), 1.88-2.25 (m, 12H), 1.80 (br t, J=13.5 Hz, 2H).

### 2(d) cis-{-4-cyano-4-[3-(trans-3-hydroxycyclopentyloxy)-4-methoxyphenyl]cyclohexane-1-carboxylic acid}

The compound of Example 2(c) (0.10 g, 0.27 mmol) was dissolved in 5:5:2 tetrahydrofuran/methanol/water (5 mL), sodium hydroxide (0.035 g, 0.88 mmol) was added and the mixture was stirred at RT for 3 h. The solvent was evaporated, the residue was partitioned between 5% aqueous NaOH and dichloromethane and the layers were separated. The aqueous layer was acidified to pH 3 with 3N aqueous hydrochloric acid and was extracted three times with 5% methanol in chloroform. The organic extracts were combined, were dried (MgS04), filtered and evaporated. The residue was purified by flash chromatography (silica gel, 90:10:1 chloroform/methanol/water) to yield a solid which was slurried in ether, was collected by filtration and was dried in vacuo to afford the title compound. MS(Cl/NH₃) m/e 377 [M + NH₃]+; ¹H NMR(400 MHz, CDCl₃) □ 7.08 (br s, 1H), 7.03 (br d, J=8.5Hz, 1H), 6.88 (d, J=8.5 Hz, 1H), 4.98 (m, 1H), 4.38 (m, 1H), 3.84 (s, 1H), 2.41 (m, 1 H), 1.77-2.29 (m, 16H); Anal. (C₂₀H₂₅NO₅•0.9 H₂O) calcd: C, 63.95; H,7.19; N,3.73. found: C, 64.06; H, 6.88; N, 3.77; mp 161-163° C.

### Reference Example 3

### Preparation of cis-{4-cyano-4-[3-(cis-3-hydroxycyclopentyloxy)-4-methoxyphenyl]-cyclohexane-1-carboxylic acid}

### 3(a) Methyl cis-{-4-cyano-4-[3-(cis-3-formyloxycyclopentyloxy)-4-methoxyphenyl]-cyclohexane-1-carboxylate}

The compound of Example 2(c) (0.68 g, 1.83 mmol) was dissolved in tetrahyrofuran (20 mL) under an argon atmosphere and was treated with triphenylphosphine ( 0.96 g, 3.66 mmol) and formic acid (0.17 g, 3.66 mmol). Diethyl azodicarboxylate (0.64 g, 3.66 mmol) was added and the mixture was stirred at RT for 16 h. The solution was evaporated, ether was added and the white solid was removed by filtration. The filtrate was concentrated and the residue was purified by flash chromatography (silica gel, 65% hexane/ethyl acetate) to yield the title compound as a clear colorless oil. ¹H NMR(400 MHz, CDCl₃) □ 8.02 (s,1H), 7.0 (d of d, J=2.4, 8.2 Hz, 1H), 6.99 (d, J=2.4 Hz, 1 H), 6.87 (d, J=8.2 Hz, 1H), 5.48 (m, 1 H), 4.95 (m, 1H), 3.84 (s, 3H), 3.72 (s, 3H), 2.31-2.40 (m, 2H), 2.13-2.28 (m, 7H), 1.96-2.06 (m, 3H), 1.74-1.87 (m, 3H).

### 3(b) cis-{-4-cyano-4-[3-(cis-3-hydroxycyclopentyloxy)-4-methoxyphenyl]cyclohexane-1-carboxylic acid}

The compound of Example 3(a) (0.52 g, 1.31 mmol) was dissolved in 5:5:2 tetrahydrofuran/methanollwater (20mL), sodium hydroxide (0.32 g, 8.0 mmol) was added and the mixture was stirred at RT for 2.5 h. The solvent was evaporated and the aqueous residue was acidified to pH 1-2 with 3N aqueous hydrochloric acid. The white solid product was collected, was washed with water and was dried in vacuo to afford the title compound as a white solid. MS(CI/NH₃) m/e 377 [M + NH3]+; 1 H NMR(250 MHz, CDCl₃) □ 6.98 (m, 2H), 6.86 (d, J=8.2 Hz, 1H), 4.97 (m, 1H), 4.59 (m, 1H), 3.85 (s, 3H), 1.64-2.47 (m, 17H); mp 143-145° C.

## Claims

1. A process for preparing an acid of formula IV 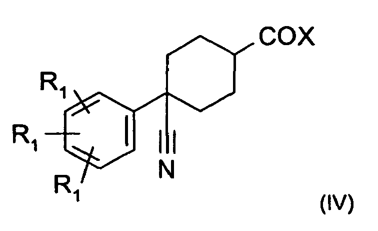 wherein R₁ is hydrogen or another substituent and X is OH or a salt thereof or -O-C₁₋₆alkyl, which process comprises hydrolyzing a thioketene of formula (C) 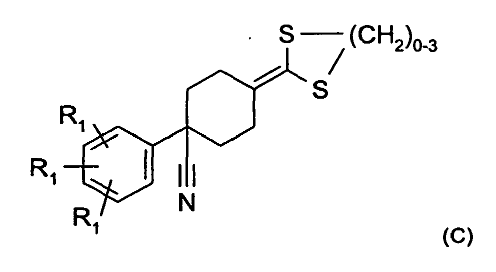 using trifluoroacetic acid and an aqueous solvent system and heating the mixture to between 40 and 80° C for 30 minutes to 2 hours, and then adding a base.

2. The process of claim 1 wherein the product is an acid of formula IV which is cis-[4-cyano-4-(3-cyclopentyloxy-4-methoxyphenyl)cyclohexane-1-carboxylic acid] or a salt thereof.

## Patentansprüche

1. Verfahren zur Herstellung einer Säure der Formel (IV) : 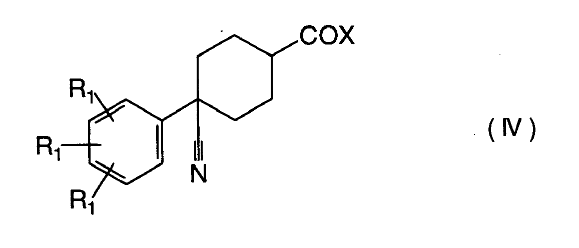 worin R₁ Wasserstoff oder ein anderer Substituent ist und X OH oder ein Salz davon oder -O-C₁₋₆-Alkyl ist, wobei das Verfahren das Hydrolysieren eines Thioketens der Formel (C) 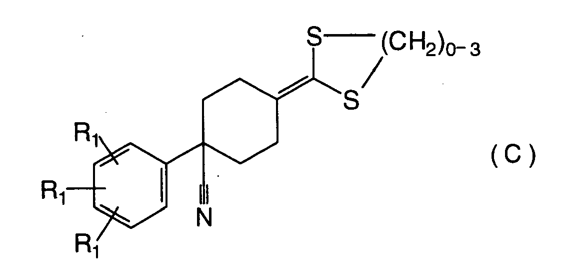 unter Verwendung von Trifluoressigsäure und eines wässrigen Lösungsmittelsystems und das Erwärmen der Mischung auf 40 bis 80°C für 30 Minuten bis 2 Stunden und das anschliessende Zugeben einer Base umfasst.

2. Verfahren gemäss Anspruch 1, worin das Produkt eine Säure der Formel (IV) ist, die cis-[4-Cyano-4-(3-cyclopentyloxy-4-methoxyphenyl)cyclohexan-1-carbonsäure] oder ein Salz davon ist.

## Revendications

1. Procédé de préparation d'un acide de formule (IV) 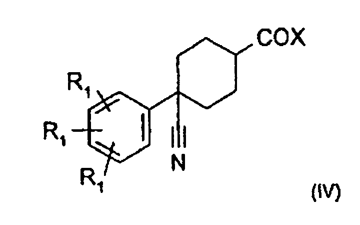 dans laquelle R₁ représente l'hydrogène ou un autre substituant, et X représente un groupe OH, ou un sel de celui-ci ou un groupe -O-alkyle C₁₋₆, lequel procédé comprend l'hydrolyse d'un thiocétène de formule (C) 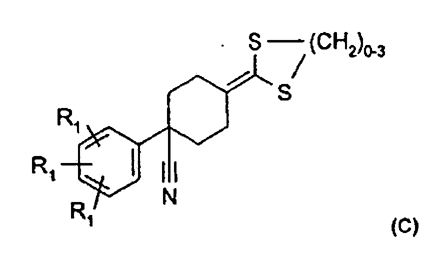 en utilisant l'acide trifluoroacétique et un système solvant aqueux et en chauffant le mélange entre 40 et 80°C pendant 30 minutes à 2 heures, et ensuite en additionnant une base.

2. Procédé selon la revendication 1, dans lequel le produit est un acide de formule (IV) qui est l'acide cis-[4-cyano-4-(3-cyclopentyloxy-4-méthoxyphényl)cyclohexane-1-carboxylique] ou un sel de celui-ci.
